(19) Europäisches Patentamt
European Patent Office
Office européen des brevets

(11) Veröffentlichungsnummer: **0 185 227**
**A2**

(12) # EUROPÄISCHE PATENTANMELDUNG

(21) Anmeldenummer: **85114963.3**

(22) Anmeldetag: **26.11.85**

(51) Int. Cl.[4]: **C 07 C 143/83**

(30) Priorität: **06.12.84 JP 256546/84**

(43) Veröffentlichungstag der Anmeldung:
**25.06.86 Patentblatt 86/26**

(84) Benannte Vertragsstaaten:
**BE CH DE FR GB IT LI NL**

(71) Anmelder: **NIHON TOKUSHU NOYAKU SEIZO K.K.**
**No.4, 2-Chome, Nihonbashi Honcho**
**Chuo-ku Tokyo 103(JP)**

(72) Erfinder: **Saito, Junichi**
**3-7-12, Osawa**
**Mitaka-shi Tokyo(JP)**

(72) Erfinder: **Tamura, Tatsuo**
**1-7-30, Hanenaka Hamura-machi**
**Nishitama-gun Tokyo(JP)**

(74) Vertreter: **Schumacher, Günter, Dr. et al,**
**c/o Bayer AG Konzernverwaltung RP Patentabteilung**
**D-5090 Leverkusen 1 Bayerwerk(DE)**

(54) **Verfahren zur Herstellung von Phenyl-N-(2-biphenylylsulfonyl)Carbamat.**

(57) Die vorliegende Erfindung betrifft ein Verfahren zur Herstellung von Phenyl-N-(2-biphenylysulfonyl) carbamat der allgemeinen Formel

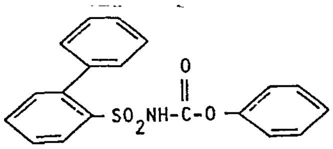

(I)

durch Reaktion von 2-Phenylbenzolsulfonamid der allgemeinen Formel

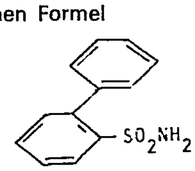

(II)

mit Diphenylcarbonat der allgemeinen Formel

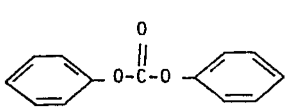

(III),

das dadurch gekennzeichnet ist, daß die Reaktion in Gegenwart eines Alkalimetallhydroxids und eines aprotischen Lösungsmittels bei Temperaturen zwischen etwa 20°C und etwa 60°C durchgefuhrt wird.-

Die Verbindung (I) kann als Zwischenprodukt zur Herstellung von bekannten herbiziden Wirkstoffen verwendet werden.

EP 0 185 227 A2

0185227

NIHON TOKUSHU NOYAKU SEIZO K.K., Tokyo/Japan

Bi-Ma
IVa/ZP

# Verfahren zur Herstellung von Phenyl-N-(2-biphenylylsulfonyl)carbamat

Die vorliegende Erfindung betrifft ein verbessertes Verfahren zur Herstellung des bekannten Phenyl-N-(2-biphenylylsulfonyl)carbamats durch Reaktion von 2-Phenylbenzolsulfonamid mit Diphenylcarbonat unter bestimmten speziellen Reaktionsbedingungen.

Der Stand der Technik (JP-Patentanmeldung 70909/1984) offenbart ebenfalls ein Verfahren zur Herstellung des genannten Phenyl-N-(2-biphenylylsulfonyl)carbamats durch Reaktion von 2-Phenylbenzolsulfonamid mit Diphenylcarbonat in Gegenwart einer Base und eines polaren Lösungsmittels unter Inertgas-Atmosphäre. In diesem Verfahren des Standes der Technik wurden als polares Lösungsmittel Dimethylformamid und als Base Natriumhydrid in der Reaktion eingesetzt, die weiterhin unter Stickstoff-Gas-Atmosphäre durchgeführt wurde.

Die Reaktion dieses Verfahrens nach dem Stand der Technik verläuft in relativ wirksamer Weise, wenn sie als Reaktion in kleinem Maßstab, etwa auf dem Niveau von Reaktionen im Laboratoriumsmaßstab, durchgeführt wird. Wird jedoch der Reaktionsmaßstab vergrößert, so stellt sich heraus, daß dieses Verfahren nach dem Stand

der Technik Nachteile in bezug auf die Ausbeute und die Reinheit des Produkts aufweist. Darüber hinaus ist die Rückgewinnung des Dimethylformamids nach der Reaktion schwierig, und die Handhabung des Natriumhydrids birgt Gefahren in sich, so daß für das Bedienungspersonal äußerste Vorsicht geboten ist (Entflammbarkeit an der Luft).

Aus diesem Grunde muß das bekannte Verfahren unter Stickstoff-Gas-Atmosphäre durchgeführt werden und kann damit nicht eine einfache Arbeitsweise ermöglichen, sondern bedingt in der Praxis kompliziertere Arbeitsgänge. Infolgedessen wirft das bekannte Verfahren verschiedene technische Probleme auf, wenn seine Durchführung im Produktionsmaßstab der Industrie versucht wird.

Nunmehr wurde gefunden, daß Phenyl-N-(2-biphenylylsulfonyl)carbamat der Formel (I)

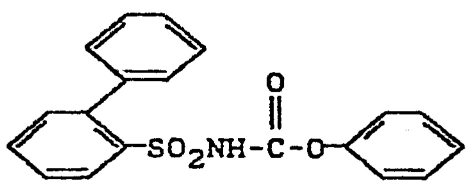

(I)

in hoher Ausbeute und Reinheit durch Reaktion von 2-Phenylbenzolsulfonamid der Formel (II)

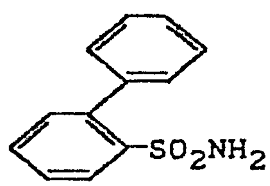

(II)

mit Diphenylcarbonat der Formel (III)

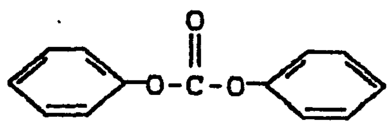

(III)

in Gegenwart eines Alkalimetallhydroxids und eines aprotischen Lösungsmittels bei Temperaturen zwischen etwa 20°C und etwa 60°C erhalten wird, ohne daß eine Inertgas-Atmosphäre zur Anwendung kommt.

Es ist als höchst überraschend zu bezeichnen, daß bei der Durchführung der Reaktion gemäß der vorliegenden Erfindung das angestrebte Produkt mit hoher Ausbeute und Reinheit selbst dann erhalten werden kann, wenn die Reaktion im technischen Maßstab durchgeführt wird, wobei nicht nur leicht zu handhabende Basen wie Kaliumhydroxid, Natriumhydroxid etc. sondern auch leicht zurückzugewinnende und wiederverwendbare Lösungsmittel wie Toluol, Xylol, Acetonitril etc. eingesetzt werden. In der Tat war im Hinblick auf den Stand der Technik in keiner Weise zu erwarten, daß es möglich sei, das aufwendige Verfahren des Standes der Technik zu vereinfachen und gleichzeitig damit auch noch wesentliche Verbesserungen in bezug auf die Reinheit, die Ausbeute, die Base und das Lösungsmittel zu erzielen, und dies sogar noch im technischen Maßstab der Industrie-Produktion, was einen wesentlichen und völlig überraschenden technischen Fortschritt gegenüber dem Stand der Technik darstellt.

Der Reaktionsablauf gemäß der vorliegenden Erfindung läßt sich beispielsweise anhand der folgenden Gleichung aufzeigen:

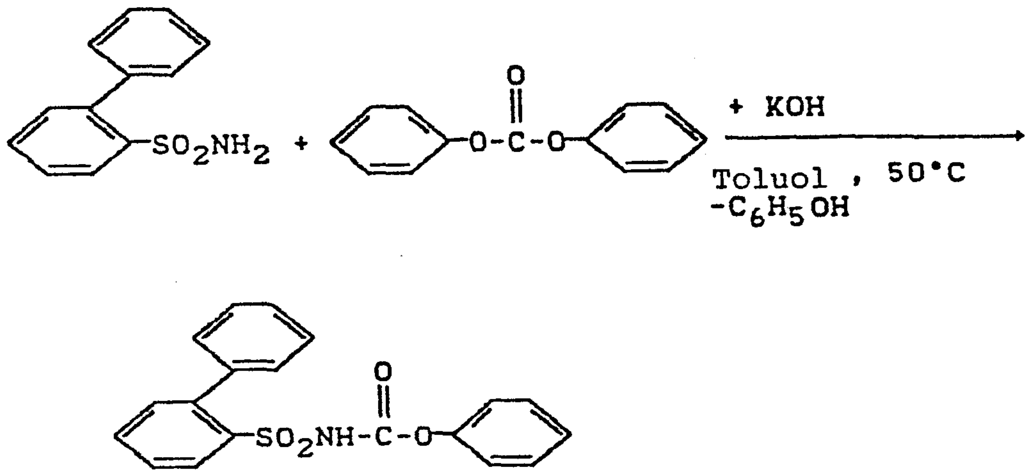


In dem vorstehenden Verfahren können als Lösungsmittel aprotische, unpolare Lösungsmittel wie beispielsweise Cyclohexan, Toluol, Xylol und Methylenchlorid, und vorzugsweise Toluol, eingesetzt werden.

Auch Acetonitril ist als aprotisches Lösungsmittel zu nennen, wenngleich es polarer Natur ist.

Als in dem vorstehenden Verfahren verwendbare Alkalimetallhydroxide sind beispielsweise Kaliumhydroxid, Natriumhydroxid und Lithiumhydroxid zu nennen, von denen Kaliumhydroxid und Natriumhydroxid bevorzugt werden.

Die Menge des in dem vorstehenden Verfahren verwendeten Alkalimetallhydroxids kann derjenigen des Ausgangsmaterials 2-Phenylbenzolsulfonamid (II) äquimolar sein (1 mol) oder bis zu etwa 1,5 mol pro 1 mol des letzteren betragen; vorzugsweise verwendet man 1,05 bis 1,2 mol Alkalimetallhydroxid pro 1 mol (II).

Das Verfahren gemäß der vorliegenden Erfindung kann bei einer Reaktionstemperatur von etwa 20°C bis etwa 60°C, vorzugsweise von etwa 30°C bis etwa 50°C und besonders

bevorzugt von etwa 40°C bis etwa 50°C, durchgeführt werden.

Das vorliegende Verfahren kann selbstverständlich unter normalem Druck durchgeführt werden, jedoch ist es auch möglich, unter erhöhtem oder vermindertem Druck zu arbeiten, wenngleich normaler Druck vorzuziehen ist.

Bei der Durchführung des Verfahrens gemäß der vorliegenden Erfindung werden auf 1 mol 2-Phenylbenzolsulfonamid (II) 1 bis 1,5 mol Diphenylcarbonat (III), entweder in einem aprotischen apolaren Lösungsmittel wie Toluol oder in einem aprotischen polaren Lösungsmittel wie Acetonitril in Gegenwart eines Alkalimetallhydroxids (in äquimolarer Menge bis zu etwa 1,5 mol auf 1 mol 2-Phenylbenzolsulfonamid (II)) bei Temperaturen von etwa 20°C bis etwa 60°C eingesetzt. Danach wird das Reaktionsprodukt zur Rückgewinnung des Lösungsmittels abfiltriert, und der Reaktionsrückstand wird in Wasser gelöst, und seine wäßrige Lösung wird anschließend durch Zusatz von Salzsäure angesäuert, wonach das gewünschte Phenyl-N-(2-biphenylylsulfonyl)carbamat (I) erhalten wird.

Das nach dem Verfahren gemäß der vorliegenden Erfindung hergestellte Phenyl-N-(2-biphenylylsulfonyl)carbamat wird vorzugsweise als Zwischenprodukt für die Herstellung herbizid wirksamer Verbindungen wie beispielsweise N-2-Biphenylylsulfonyl-N'-(4,6-dimethoxy-1,3,5-triazin-2-yl)harnstoff (vgl. die EP-A- 0 056 969) verwendet.

Der technische Gehalt der vorliegenden Erfindung wird konkret anhand der folgenden Beispiele näher beschrieben, die jedoch nicht als Einschränkung der Erfindung aufzufassen sind.

Beispiel 1

2,33 kg (10 mol) 2-Phenylbenzolsulfonamid (II), 0,59 kg (10,5 mol) Kaliumhydroxid und 2,14 kg (10 mol) Diphenylcarbonat (III) wurden zu 30 l Toluol gegeben und unter Rühren etwa 6 h bei 50°C zur Reaktion gebracht, wodurch das Kaliumsalz des angestrebten Produkts erzeugt wurde. Nach Beendigung der Reaktion wurde das Reaktionsprodukt abgekühlt und unter Saugen abfiltriert.

Die dabei erhaltenen Kristalle wurden in Wasser gelöst, und die Lösung wurde durch Zusatz von Salzsäure zu derselben angesäuert, um das gewünschte Phenyl-N-(2-biphenylylsulfonyl)carbamat (I) zu fällen. Das ausgefällte Produkt wurde mit Methylenchlorid extrahiert, und die erhaltene organische Phase wurde einige Male mit Wasser gewaschen, mit Natriumsulfat getrocknet und dann zur Entfernung des Methylenchlorids der Destillation unter vermindertem Druck unterworfen. Danach wurden 3,4 kg des gewünschten Phenyl-N-(2-biphenylylsulfonyl)carbamats (I) mit einem Schmp. 115-118°C erhalten.

Die Ausbeute, Reinheit und Netto-Ausbeute betrugen 96 %, 95 % bzw. 90,6 %.

Vergleichsbeispiel 1
(gemäß der JP-Patentanmeldung 70909/1984)

Zu 20 l getrocknetem Dimethylformamid wurden 0,252 kg (10,5 mol) Natriumhydrid unter einem Stickstoff-Gas-Strom hinzugefügt, gefolgt von der Zugabe von 5 l einer Lösung von 2,33 kg (10 mol) 2-Phenylbenzolsulfonamid

(II) in Dimethylformamid bei einer Temperatur von nicht mehr als 10°C. Das Reaktionsprodukt wurde 1 h gerührt, und danach wurden 2,14 kg (10 mol) Diphenylcarbonat (III) bei Raumtemperatur zugegeben, und die Reaktionsmischung wurde weiter 1 h bei Raumtemperatur gerührt.

Nach dem Eintragen des gesamten Volumens der Reaktionsmischung in Eiswasser wurde mit Salzsäure angesäuert und mit Ethylacetat extrahiert. Die erhaltene organische Phase wurde getrocknet und das Ethylacetat wurde aus ihr abdestilliert, wonach 2,4 kg Phenyl-N-(2-biphenylylsulfonyl)carbamat (I) erhalten wurden.

Die Ausbeute, Reinheit und Netto-Ausbeute betrugen 68 %, 81 % bzw. 55,1 %.

In der folgenden Tabelle 1 sind Ergebnisse von weiteren Umsetzungen nach dem Verfahren gemäß der vorliegenden Erfindung aufgeführt. Dabei wurden in den Beispielen 2 bis 5 unter Anwendung der gleichen Arbeitsweise, wie sie in Beispiel 1 beschrieben ist, die Reaktionsbedingungen variiert. Beispiel 6 ist ein weiteres Vergleichsbeispiel, in dem bei einer höheren Reaktionstemperatur (70°C) gearbeitet wurde.

Tabelle 1

| Beispiel Nr. | Reaktionspartner (II) + (III) | Base | Lösungsmittel | Reaktions-Temperatur und -Zeit | Menge Produkt (I) | Ausbeute (I) | Reinheit (I) | Netto-Ausbeute (I) |
|---|---|---|---|---|---|---|---|---|
| 2 (Vorlieg. Erfind.) | 2-Phenylbenzolsulfonamid 2,33 kg (10 mol) Diphenylcarbonat 2,14 kg (10 mol) | Kaliumhydroxid 0,59 kg (10,5 mol) | Acetonitril | 40°C/6 h | 3,3 kg | 93,5 % | 95 % | 88,8 % |
| 3 (Vorlieg. Erfind.) | 2-Phenylbenzolsulfonamid 2,33 kg (10 mol) Diphenylcarbonat 2,57 kg (12 mol) | Natriumhydroxid 0,48 kg (12 mol) | Toluol | 50°C/5 h | 3,4 kg | 96 % | 96 % | 92,2 % |
| 4 (Vorlieg. Erfind.) | 2-Phenylbenzolsulfonamid 2,33 kg (10 mol) Diphenylcarbonat 3,21 kg (15 mol) | Kaliumhydroxid 0,6 kg (10,7 mol) | Toluol | 50°C/5 h | 3,2 kg | 90,7 % | 91 % | 82,5 % |
| 5 (Vorlieg. Erfind.) | 2-Phenylbenzolsulfonamid 2,33 kg (10 mol) Diphenylcarbonat 2,57 kg (12 mol) | Kaliumhydroxid 0,59 kg (10,5 mol) | Xylol | 50°C/6 h | 3,4 kg | 96 % | 95 % | 90,6 % |
| 6 (Vergl.-Beispiel) | 2-Phenylbenzolsulfonamid 2,33 kg (10 mol) Diphenylcarbonat 2,14 kg (10 mol) | Kaliumhydroxid 0,59 kg (10,5 mol) | Toluol | 70°C/5 h | Spuren-Menge | <10 % | - | - |

## P a t e n t a n s p r ü c h e

1. Verfahren zur Herstellung von Phenyl-N-(2-biphenylyl-sulfonyl)carbamat der Formel (I)

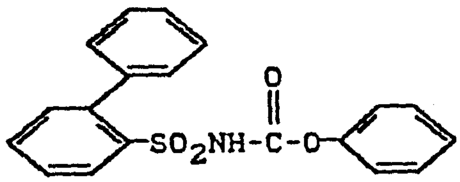

(I)

durch Reaktion von 2-Phenylbenzolsulfonamid der Formel (II)

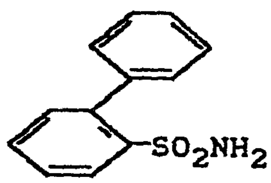

(II)

mit Diphenylcarbonat der Formel (III)

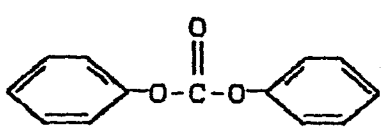

(III)

in Gegenwart einer Base und eines Lösungsmittels, dadurch gekennzeichnet, daß die Reaktion in Gegenwart eines Alkalimetallhydroxids und eines aprotischen Lösungsmittels bei Temperaturen zwischen etwa 20°C und etwa 60°C durchgeführt wird.

Nit 186

2. Verfahren nach Anspruch 1, dadurch gekennzeichnet, daß das Alkalimetallhydroxid Kaliumhydroxid oder Natriumhydroxid ist.

3. Verfahren nach Anspruch 1, dadurch gekennzeichnet, daß das aprotische Lösungsmittel ein apolares Lösungsmittel ist.

4. Verfahren nach Anspruch 1 oder 3, dadurch gekennzeichnet, daß das aprotische, apolare Lösungsmittel ein Lösungsmittel ausgewählt aus der aus Cyclohexan, Toluol, Xylol und Methylenchlorid bestehenden Gruppe ist.

5. Verfahren nach Anspruch 1 oder 3, dadurch gekennzeichnet, daß das aprotische, apolare Lösungsmittel Toluol ist.

6. Verfahren nach Anspruch 1, dadurch gekennzeichnet, daß das aprotische Lösungsmittel ein polares Lösungsmittel wie Acetonitril ist.

7. Verfahren nach Anspruch 1, dadurch gekennzeichnet, daß die Reaktionstemperatur etwa 30°C bis etwa 50°C beträgt.

8. Verfahren nach Anspruch 1, dadurch gekennzeichnet, daß die Reaktionstemperatur etwa 40°C bis etwa 50°C beträgt.

9. Verfahren nach Anspruch 1, dadurch gekennzeichnet, daß auf 1 mol 2-Phenylbenzolsulfonamid (II) 1 bis 1,5 mol Diphenylcarbonat (III) und 1 bis 1,5 mol, vorzugsweise 1,05 bis 1,2 mol, Alkalimetallhydroxid eingesetzt werden.